Europäisches Patentamt

**European Patent Office**

**Office européen des brevets**

(11) Publication number: **0 094 231**
**A1**

## EUROPEAN PATENT APPLICATION

(21) Application number: **83302596.8**

(22) Date of filing: **09.05.83**

(51) Int. Cl.³: **A 61 M 11/02**
// F04B33/00, A61M16/00

(30) Priority: **08.05.82 GB 8213356**
**13.07.82 GB 8220351**

(43) Date of publication of application: **16.11.83**
**Bulletin 83/46**

(84) Designated Contracting States: **AT BE CH DE FR IT LI LU NL SE**

(71) Applicant: **E.J. PRICE (DEVELOPMENTS) LIMITED,**
**71 Melchett Road Birmingham Factory Centre, Kings Norton Birmingham B30 3HL (GB)**

(72) Inventor: **Price, Ernest James, Regent House Pool Head Lane Wood End, Tanworth-in-Arden Solihull (GB)**

(74) Representative: **Wallbank, Roger Wickham et al, BARKER, BRETTELL & DUNCAN 138 Hagley Road, Edgbaston Birmingham, B16 9PW (GB)**

(54) **Nebulizers.**

(57) A nebulizer has a nebulizing head (24) and is supplied with compressed air from a pump which is powered by the exertions of an individual, who may be the user. The pump incorporates a cylinder (4) with a reciprocable piston (5) which generates compressed air on one stroke but not on the reverse stroke. An accumulator (19) receives compressed air from the pump and enables the head to produce a continuous spray. The accumulator may include a fine filter (26). The pump may resemble a foot-pump for inflating vehicle tyres. The piston may carry a resilient annular seal which is lightly compressed in the cylinder but is not compressed by engagement with the piston. The seal may be unlubricated. The pump is stabilized by a removable stabilizer with laterally projecting limbs (36).

FIG. 1.

1

NEBULIZERS

This invention relates to nebulizers.

Nebulizers are used for converting liquids into fine sprays, principally for medical purposes. Patients suffering from asthma, for example, often obtain relief by inhaling a fine spray of a suitable liquid. It may also be possible for vaccines to be administered orally in the form of fine sprays.

One conventional type of nebulizer comprises a nebulizing head and an electrically powered air pump operative to supply pressurized air to the head. That type of nebulizer suffers from the disadvantages that the air pump is relatively expensive, requires a source of electricity to enable it to be operated and tends to be unreliable. An aim of the present invention is to provide a nebulizer such that those disadvantages are largely or wholly avoided.

From one aspect the present invention consists in a nebulizer comprising a nebulizing head and a pump that can be operated by physical movement of an individual and is operative to supply pressurized air to the nebulizing head, the pump incorporating a cylinder with a reciprocable piston so arranged that in use pressurized air for the head is generated on one stroke of the piston but not on the reverse stroke thereof, there being an accumulator for the pressurized air such that in use it is possible for the head to produce a continuous spray of liquid.

The pump preferably comprises a base and an operating member pivoted together at their ends and a piston-and-cylinder unit coupled to the base and operating member in such a manner that as the operating member is depressed from a raised position the unit performs a stroke of one kind and as the operating member returns to its raised position the unit performs a stroke of the other kind. A conventional foot pump for use in inflating the tyres of motor vehicles is a pump of that kind. The pump preferably includes spring means operative to return the operating member to its raised position. The spring means may be incorporated in the piston-and-cylinder unit. The pump is preferably such that it is the reverse stroke of the pump that is effected by the spring means; thus the arrangement is preferably such that when the operating member is depressed the unit generates pressurized air for the head. Nevertheless the reverse arrangement could be adopted if desired, in which case the spring means would bring about the generation of pressurized air for the head.

A simple experiment can be made to determine whether the accumulator is of sufficient size. The piston in the pump is caused to reciprocate through its full working travel and the nebulizing head is observed. Provided a continuous spray is generated by the head, the accumulator is large enough. One stroke of the pump is a pressure-generating stroke, and the other stroke is a return stroke, when no pressure is generated. As indicated above it is normal for the pressure-generating stroke to be that caused by the application of force by the user and for the return stroke to be effected by spring means. In that case, the length of the period of time taken for the return stroke is dependent on the physical properties of the pump. The pump is preferably such that the time taken for the spring means to effect the reverse stroke is no more than three seconds. The length of that period of time may well be less than three seconds. It may in fact be no more than two seconds, and could well be as short as one second or even less.

The pump may be operated by the user's foot or, if the pump is placed on a table or in some other suitable position, it may be operated by the user's hand or in some other way by the user. The pump may be provided with stabilizing means comprising one or more ground-engaging limbs extending laterally outwards from the base of the pump. The limb or each limb is preferably retractable or removable so as to enable the pump to be made more compact for storage and transport than it is when the stabilizing means is in its position of use.

The nebulizing head may well be such as to operate satisfactorily with any air pressure within a range of pressures. The lower end of that range may, for example, be no greater than 5 p.s.i. (0.34 bars). Alternatively the lower end of that range may be no greater than 10 p.s.i. (0.69 bars). It is therefore necessary to ensure that the accumulator is sufficiently large to cause the pressure of the air supplied to the head to remain above the lower end of the range. The upper end of the range may be between 10 p.s.i. and 20 p.s.i. (0.69 bars and 1.37 bars) or even higher. Further, it is desirable to provide means such as to make it unlikely or impossible for the pressure of the air supplied to the head to rise above a predetermined upper limit. To this end there may be a pressure-relief valve which opens to allow air to escape when that pressure limit is exceeded, or the nebulizer may be so constructed that part of it becomes disconnected if that pressure limit is exceeded. Alternatively, or in addition, a restrictor may be incorporated in the path of the pressurized air to the head. Nevertheless, it is found experimentally that in

4

practice, whether or not any of these means is provided, it is easy for the user to operate the pump in such a manner that there is a steady continuous spray emanating from the nebulizer head.

It is most desirable that the pump should be free of any liquid lubricant or other lubricant that might be introduced into the pressurized air. Hitherto grease or silicone oil has often been used to lubricate the seal in a pump of the conventional kind briefly described above. It is now preferred to avoid the need for lubricating the seal. There is preferaby a seal on the piston comprising an annular body of resilient material that is resiliently deformed by its engagement with the interior of the cylinder and is located, inasfar as its radial position is concerned, solely by its engagement with the interior of the cylinder. Hitherto it has generally been the practice to locate the seal in a circumferential groove in the piston, the groove being of a depth such that the seal is slightly compressed between the base of the groove and the bore of the cylinder. It has now been found, however, that it is advantageous to avoid any radial compression of the seal from the base of the groove.

The seal is preferably of such dimensions that while it is slightly deformed when fitted into the cylinder it is less deformed than is customary with the seals of such pumps. Thus the outside diameter of the seal (when unrestricted) is preferably no more than 0.02 inches (0.508 mm) greater than the inside diameter of the cylinder, a typical value being 0.015 inches (0.381 mm).

The piston may be positively located in the cylinder so that it is co-axial with the cylinder at all times. This, however, is not essential, and the piston may be free to move laterally, at least to a small extent, so that it is not strictly co-axial with the cylinder. In a preferred arrangement the piston is free laterally in the cylinder but the lateral movement of the piston is restricted so that the piston cannot touch the bore of the cylinder, that part of the piston inside the seal being of such a shape and dimensions that on lateral movement of the piston in the cylinder said part of the piston abuts an internal face of the seal before the piston can engage the bore of the cylinder.

Similarly, it is preferred to locate the seal on the piston in such a manner that it is not compressed in the longitudinal direction of the piston, that is in the direction of the reciprocal movement of the piston.

The only permanent constraint applied to the seal, therefore, is that applied by the bore of the cylinder itself, of which the internal dimensions are smaller than the external dimensions of the seal when the seal is free to assume its natural, unconstrained shape.

The seal is preferably located axially of the piston between two end stops constituting part of the piston; these are preferably so spaced apart as to permit some relative axial movement between the seal and the piston. At least one of the end stops preferably presents a planar face for sealing engagement with the seal when that face moves in the direction of the seal, so as to bear against the seal. The two end stops may present parallel planar faces for sealing engagement.

The seal is preferably made of a material that is softer than that customarily used for the seals of pumps of the kind described, a satisfactory material being a rubber or rubber-like material having a Shore hardness of

0094231

6

about 38. The seal may be coated with a substance having a coefficient of friction lower than that of the material from which the seal is formed; a suitable substance is p.t.f.e. (polytetrafluorethylene). The p.t.f.e. is preferably applied to the ring by a spraying process, with a resin carrier and a solvent, the layer thus formed on the ring being baked to cause the solvent to evaporate and the resin to set. It is found that within limits the resultant coating of p.t.f.e. increases in hardness with increased baking and that within limits the adherence of the coating increases with increased baking. It is therefore preferred to bake the layer for longer than is customary in the normal use of this coating process.

The seal is preferably of circular cross-section, thus constituting an O-ring.

The bore of the cylinder may be formed from any suitable material, though it is preferred for the bore to be very smooth. The cylinder may present a metallic surface for engagement by the seal, or the surface may be of a plastics material.

While the seal may operate satisfactorily in the absence of lubricants, the use of lubricants is not precluded. Similarly, while the seal is preferably used in a dry state, the presence of a liquid or liquids is not precluded. In particular, where the piston-and-cylinder unit is used with air containing moisture and oil in suspension the seal may still operate satisfactorily.

The accumulator preferably comprises a chamber or chambers in which sufficient pressurized air can be accumulated to ensure that the head continues to operate during a full return stroke of the piston in the cylinder. The accumulator is preferably provided with an inlet and an outlet separate from the inlet, the arrangement being such that in use pressurized air is constrained to pass from the pump, through the inlet into the accumulator and through the outlet to the nebulizing head. Nevertheless it would be possible to provide an accumulator with a common inlet and outlet, connected at a T-junction to the principal air passage from the pump to the nebulizing head. Where the accumulator comprises two or more chambers they may be arranged in a combination of these ways. Where there are a plurality of chambers,· each with an inlet and an outlet, the chambers may be arranged in series or in parallel or in a more complex arrangement.

In order to prevent the return of pressurized air into the pump, it is normally necessary to provide a non-return valve between the pump and the accumulator.

There is preferably a filter between the pump and the nebulizing head, the filter being operative to prevent unwanted material, such as dust or other particles of matter, reaching the inlet of the head. The filter may be in the form of a layer or layers of pervious sheet material, or it may be in the form of a sponge-like material, the pores of which are intercommunicating. The filter is preferably sufficiently fine to prevent matter of a dimension larger than one micron passing through it. Such a filter may act to prevent the passage of bacteria through it. In a preferred arrangement of the kind in which the accumulator is such that pressurized air from the pump is constrained to pass through it to the nebulizing head, the accumulator comprises a chamber of cross-sectional area greater than that of the remainder of the air duct between the pump and the nebulizing head, and a filter extends across the

8

the interior of the chamber. In this way it is possible to provide a filter having the same or substantially the same cross-sectional area as that of the interior of the accumulator. The resistance to air-flow afforded by the filter is thus likely to be less than that afforded by a filter disposed in the remainder of the air duct. Further, owing to the increased cross-sectional area, the rate at which the filter is likely to become clogged is correspondingly reduced. Where the accumulator comprises a plurality of chambers, filter material may be disposed in more than one such chamber; where the chambers are disposed in parallel, such an arrangement may be essential to ensure that all the air passing to the nebulizing head passes through at least one filter. The accumulator may be mounted on the pump, while a length of flexible piping may be connected between the accumulator and the nebulizing head.

Alternatively the accumulator may merely comprise a length of piping, preferably flexible piping, of sufficient volume, connected between the pump and the nebulizing head. If this latter expedient is used, the piping is preferably of an internal diameter greater than that normally used with conventional foot pumps. It would, of course, be possible to combine these two principles and to provide an accumulator comprising a chamber or chambers and a length of piping of an internal diameter greater than that normally used with foot pumps.

It is to be understood that in some circumstances the patient may operate the pump but that in others the pump will be operated by a doctor or other person attending the patient.

From another aspect the present invention consists in the combination of a pump and an accumulator for use in conjunction with a nebulizing head to form a nebulizer in accordance with that aspect of the invention set out above. The combination may include any of the preferred features also set out above.

It has been found that a preferred form of pump outlined above is suitable for use not only in supplying compressed air for a nebulizing head but also for other purposes as well.

From another aspect, therefore, the present invention consists in a pneumatic piston-and-cylinder unit comprising a cylinder containing a reciprocable piston and a seal carried by the piston for sealing engagement with the bore of the cylinder, the seal comprising a ring of resilient material of outside dimensions slightly greater than the inside dimensions of the cylinder so that the ring is resiliently deformed, the ring extending around part of the piston that is of a shape and dimensions such that the ring is not compressed between that part of the piston and the bore of the cylinder. The unit is preferably incorporated in a pump of the kind outlined above. It is also to be understood that the unit may incorporate any of the preferred or optional features referred to herein in connection with the pump that forms part of the nebulizer described herein.

One particular form of nebulizer embodying the present invention will now be described in more detail, by way of example, with reference to the accompanying drawings, in which:-

Figure 1 is a side view of the nebulizer,

*Figure 2* is a section, to a larger scale, through an accumulator constituting part of the nebulizer shown in Figure 1,

*Figure 3* is a longitudinal section through part of a pneumatic piston and cylinder unit shown in Figure 1, to a larger scale than Figure 1,

*Figure 4* is a section along the line 4-4 of Figure 1, to a larger scale than Figure 1, and illustrates a stabilizer incorporated in the nebulizer,

*Figure 5* is an end view of the stabilizer shown in Figure 4, but to a smaller scale than Figure 4, and

*Figure 6* is a section of an optional attachment for the nebulizer.

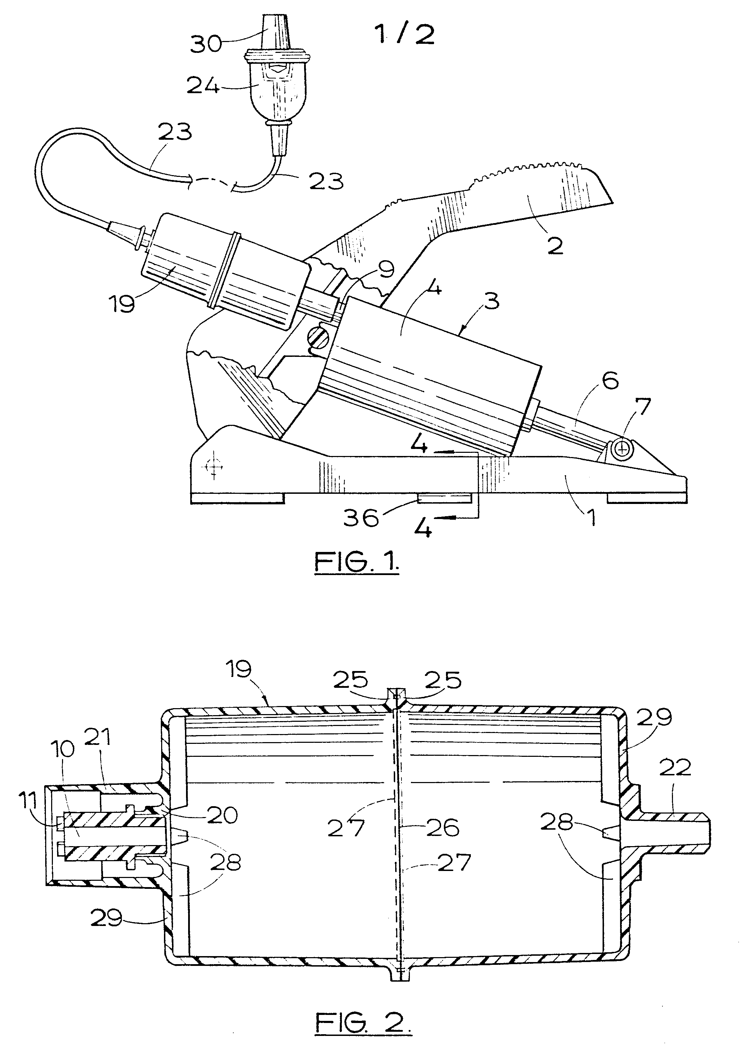

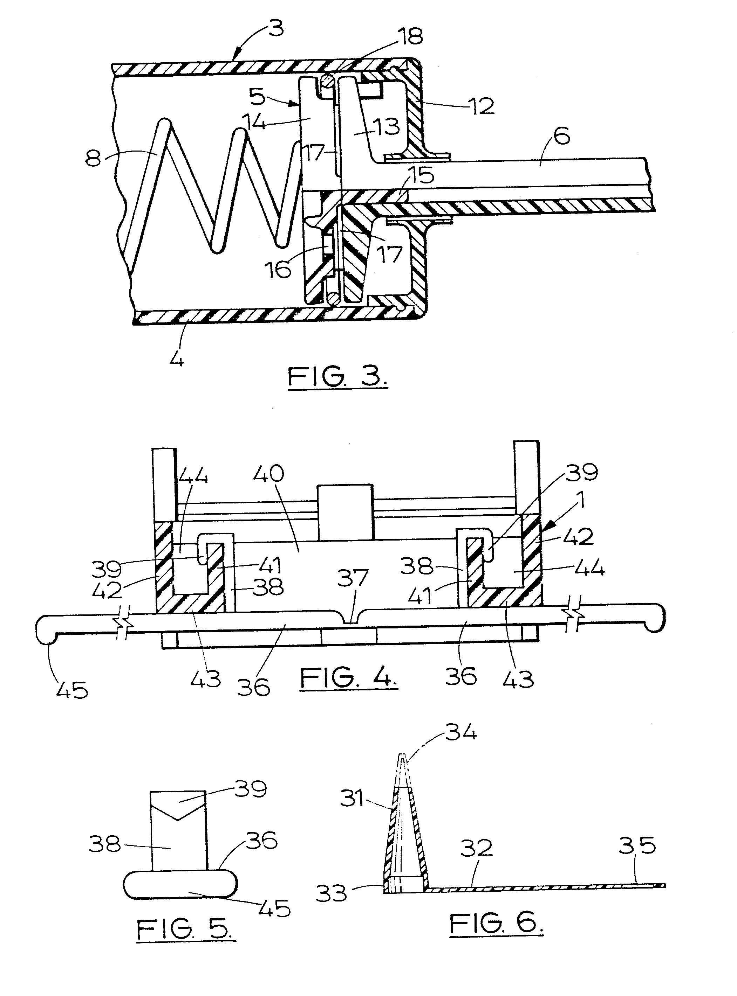

The nebulizer illustrated includes a pump largely similar to the foot-pump described and illustrated in British patent specifications Nos. 1 603 302 and 1 603 303 of E.J. Price (Developments) Limited. The pump comprises a base 1 and a treadle or operating member 2 pivoted together at their ends. A piston-and-cylinder unit 3 comprises a cylinder 4, pivoted to the treadle, and a piston 5 (Figure 3) with a piston-rod 6 pivoted to the base at 7. The cylinder 4 also houses a compression spring 8 which biases the piston to a position in which the piston-rod projects to its maximum extent, and thus biases the treadle 2 to a raised position, as shown in Figure 1. Depression of the treadle 2 from that raised position, which can be effected by the user's foot or hand or otherwise, causes the piston 5 to expel air from the cylinder 4. Release of the treadle 2 allows the spring 8 to bring about a reverse stroke of the piston during which air is not expelled from the cylinder.

Air leaves the cylinder through a tubular outlet 9 parallel with the axis of the cylinder and projecting from that end of the cylinder further from the piston-rod. At the inner end of the tubular outlet is a pierced plate (not shown), integral with the end of the cylinder. A resilient disc (not shown) is contained in the tubular outlet 9 and constitutes the valve member of a non-return valve. One end portion of a tubular connector 10 (Figure 2) is a tight fit in the tubular outlet 9. As shown at 11, the inner end face of the connector is castellated; and that castellated end is spaced from the pierced plate. In use, air expelled from the cylinder 4 displaces the valve member from the pierced plate and passes between the castellations 11 and through the connector 10. On the reverse stroke, any back-pressure in the connector 10 forces the valve member into sealing engagement with the pierced plate and thus prevents air returning into the cylinder.

The cylinder 4 comprises a moulding of plastics material with a smooth bore. Its rear end is closed by a circular end cap 12 having a central hole through which the piston rod 6 extends. The end cap 12 may be formed with an air inlet aperture, but in the preferred arrangement illustrated the central hole is rather larger in diameter than the piston rod 6 so as to allow air to enter the cylinder through the hole.

The piston 5 is of composite construction, having a rear plate 13 and a front plate 14. Both of those plates are of circular outline and are rather smaller in diameter than the bore of the cylinder 4. The rear plate 13 is formed integrally with the piston rod 6. A cylindrical bore extends axially through the piston rod and the rear plate. The front plate 14 forms part of a plastics moulding which also includes an axial spigot 15 which is secured within an end portion of the bore of the piston rod 6. Four air holes 16 (only one of which is visible in Figure 3) extend through the front plate 14, and their rear ends communicate with a gap left between the front and rear plates. Four radially extending ribs 17, formed on the rear face of the front plate 14 between the air holes 16, engage the rear plate 13, and their height determines the axial thickness of the gap between the plates. · The rear face of the front plate 14 is formed with a peripheral step which defines part of a circumferential groove in the piston. The groove is of approximately square cross-section, and the base of the groove communicates with the gap between the plates 13 and 14.

An annular seal 18 of circular cross-section is disposed in the groove. The dimensions of the seal are such that it is lightly compressed by its engagement with the bore of the cylinder. The internal diameter of the seal is greater than the diameter of the base of the groove so that the ring is not compressed between the base of the groove and the bore of the cylinder. Although this is not illustrated, the arrangement may be such that if the piston moves laterally towards the bore of the cylinder the base of the groove engages the seal before the piston can touch the bore. In the arrangement illustrated the piston can engage the bore of the cylinder without being hindered by the seal. The piston is free to move laterally in the cylinder and is not prevented from so doing by the fit of the piston rod in the central hole in the end cap 12.

13

The side walls of the groove in the piston are afforded by the plates 13 and 14, and the distance between them is greater than the axial thickness of the seal. The side walls of the groove (which constitute end stops) present parallel, planar surfaces for engagement with the seal. It will be appreciated from the foregoing description that the annular seal 18 is located in a radial direction solely by the engagement between the seal and the interior of the cylinder 4, and that the seal is located axially between end stops which are spaced apart so as to permit some relative axial movement between the seal and the piston.

When the spring 8 pushes the piston 5 towards the end cap 12, the piston pulls the seal 18 along the bore of the cylinder in engagement with the front plate 14. Air from inside that part of the cylinder between the piston and the end cap 12 can pass around the rear plate 13 into the groove between the seal and the rear plate. The air can then pass from the groove, through the gap between the plates and through the air holes 16 into that part of the cylinder between the piston and the air outlet. When the treadle 2 is depressed, and the piston is pushed away from the end cap 12, the seal 18 engages the rear plate 13 and seals against it, thus preventing the return of air through the air holes and the gap between the plates. Air is therefore forced from the cylinder through the air outlet.

In one specific example, the internal diameter of the cylinder is 2.183 inches (55.45 mm) and the internal diameter of the O-ring seal on the piston is 1.934 inches (49.12 mm). The width of the seal is 0.132 inches (3.35 mm) so that the outside diameter of the seal is 0.015 inches (0.38 mm) greater than the internal diameter of the cylinder. The seal is made from a rubber or rubber-like material with a Shore hardness of 38. The seal is not lubricated but is coated with p.t.f.e. The p.t.f.e. is applied to the ring by a spraying process, with a resin carrier and a solvent, the layer thus formed on the ring being baked to cause the solvent to evaporate and the resin to set. It is found that within limits the resultant coating of p.t.f.e. increases in hardness with increased baking and that within limits the adherence of the coating increases with increased baking. It is therefore preferred to bake the layer for longer than is customary in the normal use of this coating process.

An accumulator 19 is fitted to the tubular connector 10. The accumulator comprises a substantially cylindrical housing with an integral tubular boss 20 projecting axially from its inlet end. A cylindrical shroud 21 surrounds the boss. During assembly of the nebulizer the accumulator is fitted onto the tubular connector 10, the projecting end portion of the connector being externally screw-threaded and cutting a complimentary thread in the bore of the boss 20. The shroud 21 surrounds the tubular connector and the tubular outlet 9 of the cylinder. The accumulator 19 can readily be unscrewed and replaced as desired. An axially projecting connector 22 at the other end of the accumulator enters one end of a length of flexible piping 23, the other end of which is connected to a nebulizing head 24. The housing of the accumulator 19 is made from two largely similar plastics mouldings having around their mouths outwardly directed annular flanges 25 which are welded together. The accumulator contains a filter comprising a sheet 26 of pervious material supported between a pair of discs 27 made of a woven plastics mesh. The pores of the sheet 26 are sufficiently fine to prevent the passage through the sheet of any particles greater than one micron across. During manufacture, marginal parts of the discs extend between the flanges 25; as the flanges are welded together those marginal parts also become welded to the flanges or merge with them or are permanently bonded and sealed to them. In an alternative arrangement (not illustrated) each moulding contains a filter element of sponge-like form, with intercommunicating pores. Radial ribs 28 are formed on the inner face of each of the circular end walls 29 of the housing to hold those sponge-like filter elements away from the end walls and so to permit the ready flow of air to and from the adjacent end faces of the filter elements.

A typical housing is about 2 inches (50.8 mm) in outside diameter and about 3 inches (76.2 mm) long. The volume of such an accumulator is between 7.5 and 8.0 cubic inches (123 and 131 cm³). A smaller housing may be used if desired, for example one about 1.5 inches (38.1 mm) in diameter and about 2 inches (50.8 mm) long. The volume of such a housing is between 2.5 and 3.0 cubic inches (41 and 49 cm³). If desired, two similar housings or even three similar housings may be secured together in series or in parallel to form the accumulator.

In a modification (not illustrated) the relatively narrow flexible piping 23 is replaced by flexible piping of a sufficiently large volume to act as an accumulator without the need for the inclusion of a separate accumulator housing. In a typical arangement of that kind the piping has a length of five feet (1.52 m) and internal diameter of five eights of an inch (15.9 mm), thus providing a volume of about 18.4 cubic inches (301 cm³).

It will be appreciated that a combination of these different forms of accumulator may be used, that is one or more housings of relatively small capacity and flexible piping with a capacity larger than is conventional.

The piston of a typical pump sweeps out a volume of between 13.5 and 14.0 cubic inches (221 and 229 cm³) each stroke. The pump can easily be operated at 40 cycles (that is 80 strokes) per minute, though in practice it is likely to be operated more slowly. The most suitable rate can readily be determined by experiment, and will of course depend on the nature and design of the nebulizing head. Many such heads operate satisfactorily when air is applied to them at pressures of between 5 and 20 p.s.i. (0.34 and 1.37 bars).

The nebulizing head 24 may be of any known kind. Two typical heads are those sold under the names "ACORN" and "WEE-NEB" by VIX of Jamestown, California, U.S.A. Another suitable head is marketed under the name INSPIRON "MINI-NEB" by C. R. Bard International of Sunderland, England. In each of these types of head, a jet of air entrains a stream of liquid which is forced rapidly through a small orifice and against the convex surface of a spreader adjacent to the orifice.

It is conventional practice for a nebulizing head to have a cylindrical outlet tube 30 about half an inch long and half an inch in diameter (12.7 mm) and for this outlet to be fitted into an inlet in a face mask which is applied to the patient's face and which may be held in place by a strap. To avoid the need for a face mask, and to enable the spray of liquid to be directed in a relatively narrow jet into the patient's mouth, there may be provided an attachment of the kind shown in Figure 6. This is made as a moulding of a flexible plastics material and comprises a nozzle 31 and a retaining strap 32. The nozzle is of generally conical shape, with a cylindrical portion 33 at its broader end. When the attachment is moulded, the narrower end of the nozzle either terminates in a closed point or terminates in a relatively small hole. An end portion of the nozzle can then be cut away, as indicated by the broken lines 34, to leave an outlet hole of any desired diameter. The end portion may be cut away in the factory or by the doctor attending the patient or by the patient. At that end of the strap 32 further from the nozzle 31 there is a part-circular portion 35 with a central hole. During assembly of the nebulizer an end portion of the flexible piping 23, adjacent to the nebulizing head 24, is passed through the hole. The attachment is thus held captive even when the nozzle is temporarily removed from the outlet tube 30.

The attachment may also be used for the production of a relatively fine stream of spray for external application to a limited area of a patient's skin. For example it may be used in the application of appropriate liquids, in the form of sprays, to skin burns.

In Figures 4 and 5 there is shown a stabilizer which serves to stabilize the pump and to reduce the risk of it being tipped over sideways when in use. The stabilizer is formed as a unitary moulding of a plastics material and comprises two limbs 36 in the form of rectangular strips, disposed end to end and interconnected by an integral hinge 37 of reduced thickness. Each limb 36 has a lug 38 upstanding from it and each lug has a downwardly opening hook 39 at its upper end, the hooks being on the outer sides of the lugs. A central part of the base 1 of the pump is formed with a rectangular opening 40 so that in this part the base merely comprises two spaced parallel bars, one on each side of the opening. Each bar comprises two parallel, vertical walls 41 and 42 with their lower edges interconnected by a horizontal panel 43 and with their upper parts interconnected at longitudinally spaced intervals by transverse septae 44. The limbs 36 of the stabilizer lie beneath this central part of the base 1 and extend laterally beyond the base. A ground-engaging bead 45 is formed at the outer end of each limb. The lugs 38 extend upwards into the opening 40 in the base, and the hooks 39 extend over upper edge portions of the inner walls 41 of the side bars to retain the stabilizer in place.

When the stabilizer is to be attached to the base it is folded about the hinge 37 so that the undersides of the limbs 36 are closely adjacent to each other. The stabilizer is inserted hinge first from below into the opening 40 in the base 1. The limbs are then swung apart; at the same time the hooks 39 are fitted over the inner walls 41 of the bars alongside the opening. The material from which the stabilizer is made is sufficiently flexible and resilient to accommodate the slight distortion that occurs as the stabilizer is being attached.

It will be understood that this stabilizer is of general application to pumps of the kind described, and in particular it may be used with a pump intended for use in inflating vehicle tyres.

Finally it will be appreciated that it is a relatively simple task to disassemble the entire nebulizer (apart from the accummulator 19) and to clean and sterilize the component parts thereof.

CLAIMS

1. A nebulizer comprising a nebulizing head (24) and a pump characterised in that the pump is of the kind that can be operated by physical movement of an individual and is operative to supply pressurized air to the nebulizing head, the pump incorporating a cylinder (4) with a reciprocable piston (5) so arranged that in use pressurized air for the head is generated on one stroke of the piston but not on the reverse stroke thereof, there being an accumulator (19) for the pressurized air such that in use it is possible for the head to produce a continuous spray of liquid.

2. A nebulizer according to claim 1 characterised in that the pump comprises a base (1) and an operating member (2) pivoted together at their ends and a piston-and-cylinder unit (3) coupled to the base and operating member in such a manner that as the operating member is depressed from a raised position the unit performs a stroke of one kind and as the operating member returns to its raised position the unit preforms a stroke of the other kind.

3. A nebulizer according to claim 2 characterised in that the pump includes spring means (8) operative to return the operating member to its raised position.

4. A nebulizer according to claim 3 characterised in that the pump is such that it is the reverse stroke of the pump that is effected by the spring means.

5. A nebulizer according to claim 4 characterised in that the pump is such that the time taken for the spring means to effect the reverse stroke is no more than three seconds.

6. A nebulizer according to any one of the claims 2 to 5 characterised in that it includes stabilizing means comprising one or more ground-engaging limbs (36) extending laterally outwards from the base of the pump.

7. A nebulizer according to claim 6 characterised in that the limb or each limb (36) of the stabilizing means is retractable or removable so as to enable the pump to be made more compact for storage and transport than it is when the stabilizing means is in its position of use.

8. A nebulizer according to any one of the preceding claims characterised in that the nebulizing head (24) is capable of operating satisfactorily within a range of air pressures, the lower end of that range being no greater than 5 p.s.i. (0.34 bars).

9. A nebulizer according to any one of claims 1 to 7 characterised in that the nebulizing head (24) is capable of operating satisfactorily within a range of air pressures, the lower end of that range being no greater than 10 p.s.i. (0.69 bars).

10. A nebulizer according to any one of the preceding claims characterised in that a pressure-relief valve or a restrictor or other means is provided to prevent the pressure of the air supplied to the head rising so high that the head can not operate.

11. A nebulizer according to any one of the preceding claims characterised in that the pump is free from any liquid lubricant or other lubricant that might be introduced into the pressurized air.

12. A nebulizer according to any one of the preceding claims characterised in that there is a seal (18) on the piston comprising an annular body of resilient material that is resiliently deformed by its engagement with the interior of the cylinder and is located, inasfar as its radial position is concerned solely by its engagement with the interior of the cylinder.

13. A nebulizer according to claim 12 characterised in that the outside diameter of the seal (when unrestricted) in no more than 0.02 inches (0.508 mm) greater than the inside diameter of the cylinder.

14. A nebulizer according to either of claims 12 and 13 characterised in that the piston (5) is free to move laterally in the cylinder but the lateral movement of the piston is restricted so that the piston cannot touch the bore of the cylinder, that part of the piston inside the seal (18) being of such a shape and dimensions that on lateral movement of the piston in the cylinder said part of the piston abuts an internal face of the seal before the piston can engage the bore of the cylinder.

15. A nebulizer according to any one of claims 12 to 14 characterised in that the seal (18) is located axially between two end stops (13, 14) constituting part of the piston and spaced apart so as to permit some relative axial movement between the seal and the piston.

16. A nebulizer according to any one of claims 12 to 15 characterised in that the seal (18) is made of a rubber or rubber-like material having a Shore hardness of about 38.

17. A nebulizer according to any one of claims 12 to 16 characterised in that the seal is coated with a substance having a coefficient of friction lower than that of the material from which the seal is formed.

18. A nebulizer according to any one of the preceding claims characterised in that the accumulator (19) is provided with an inlet (10) and an outlet (22) separate from the inlet, the arrangement being such that in use pressurized air is constrained to pass from the pump, through the inlet into the accumulator and through the outlet to the nebulizing head.

19. A nebulizer according to claim 18 characterised in that the accumulator (19) comprises a chamber of cross-sectional area greater than that of the remainder of the air duct between the pump and the nebulizing head, and a filter (26) extends across the interior of the chamber.

20. A nebulizer according to claim 19 characterised in that the filter (26) is sufficiently fine as to prevent matter of a dimension larger than one micron passing through it.

21. A nebulizer according to claim 18 characterised in that the accumulator comprises a length of piping, of sufficient volume, connected between the pump and the nebulizing head.

22. In combination a pump and an accumulator for use in conjunction with a nebulizing head to form a nebulizer in accordance with any one of the preceding claims.

23. A pneumatic piston-and-cylinder unit comprising a cylinder (4) containing a reciprocable piston (3) and a seal (18) carried by the piston for sealing engagement with the bore of the cylinder, the seal comprising a ring of resilient material of outside dimensions greater than the inside dimensions of the cylinder so that the ring is resiliently deformed, characterised in that the outside

23

dimensions of the seal are only slightly greater than the inside dimensions of the cylinder and that the ring extends around part of the piston that is of a shape and dimensions such that the ring is not compressed between that part of the piston and the bore of the cylinder.

0094231

1/2

FIG. 1.

FIG. 2.

FIG. 3.

FIG. 4.

FIG. 5.

FIG. 6.

0094231

European Patent
Office

**EUROPEAN SEARCH REPORT**

Application number

EP 83 30 2596

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | GB-A- 471 127 (A. PONGARCZ)  <br><br> * Whole document * <br> --- | 1-4,10 ,12,14 ,15,18 ,22,23 | A 61 M 11/02 //<br>F 04 B 33/00<br>A 61 M 16/00 |
| Y | US-A-1 923 654 (A.C. ANOREASEN)  <br><br> * Whole document * <br> --- | 1-4,10 ,12,14 ,15,18 ,22,23 | |
| D,Y | GB-A-1 603 303 (E.J. PRICE [DEVELOPMENTS] LTD.)  <br><br> Page 1, lines 10-30; page 2, lines 60-96; page 3, lines 3-9; figures * <br> --- | 2-4,12 ,14,15 ,23 | |
| Y | GB-A-1 502 309 (ASSOCIATED SPRAYERS LTD.)  <br> * Page 1, lines 53-79; figures * <br> ----- | 10 | **TECHNICAL FIELDS SEARCHED (Int. Cl. ³)** <br><br> A 61 M <br> B 05 B <br> F 04 B |

The present search report has been drawn up for all claims

| Place of search | Date of completion of the search | Examiner |
|---|---|---|
| THE HAGUE | 10-08-1983 | FERGUSON J.R. |

EPO Form 1503. 03.82